(19) Europäisches Patentamt
European Patent Office
Office européen des brevets

(11) Veröffentlichungsnummer: **0 133 711**
**B1**

(12) # EUROPÄISCHE PATENTSCHRIFT

(45) Veröffentlichungstag der Patentschrift: **17.02.88**

(51) Int. Cl.[4]: **C 07 C 49/713**, C 07 C 45/67, B 01 J 31/24

(21) Anmeldenummer: **84109625.8**

(22) Anmeldetag: **13.08.84**

(54) **Isomerisierung von 1,3,3-Trimethyl-7-oxabicyclo[4.1.0]heptan-2-on.**

(30) Priorität: **23.08.83 CH 4593/83**

(43) Veröffentlichungstag der Anmeldung: **06.03.85 Patentblatt 85/10**

(45) Bekanntmachung des Hinweises auf die Patenterteilung: **17.02.88 Patentblatt 88/7**

(84) Benannte Vertragsstaaten: **AT BE CH DE FR GB IT LI NL**

(56) Entgegenhaltungen:
**EP - A - 0 031 875**

**JOURNAL OF THE AMERICAN CHEMICAL SOCIETY, Band 102, Nr. 6, 12. März 1980; M. SUZUKI et al.: "Palladium(O)-catalyzed reaction of alpha,betha-Epoxy ketones leading to beta-diketones", Seiten 2095-2096**
**PATENT ABSTRACTS OF JAPAN, Band 4, Nr. 19 (C-73), 16. Februar 1980, Seite 26 C 73; & JP - A - 54 154 725 (TEIJIN) 12.06.1979**
**PATENT ABSTRACTS OF JAPAN, Band 5, Nr. 62 (C-52)[734], 25. April 1981; & JP - A - 56 15216 (TEIJIN) 14.02.1981 (Kat. A,D)**

(73) Patentinhaber: **F. HOFFMANN-LA ROCHE & CO. Aktiengesellschaft, CH-4002 Basel (CH)**

(72) Erfinder: **Lukàc, Teodor, Klusstrasse 32, CH-4147 Aesch (CH)**
Erfinder: **Soukup, Milan, Dr., Blumenweg, CH-4332 Stein (CH)**
Erfinder: **Widmer, Erich, Dr., Mittelweg 47, CH-4142 Münchenstein (CH)**

(74) Vertreter: **Zimmermann, Hans, Dr. et al, Grenzacherstrasse 124 Postfach 3255, CH-4002 Basel (CH)**

**EP 0 133 711 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

**Beschreibung**

Die vorliegende Erfindung betrifft ein neues Verfahren zur Herstellung von 3-Hydroxy-2,6,6-trimethyl-2-cyclohexen-1-on, einer Schlüsselverbindung in Canthaxan thinsynthesen, mittels Isomerisierung eines Epoxids.

Isomerisierungen von Epoxiden zu Carbonylverbindungen in Gegenwart von Lewis-Säuren sind seit längerer Zeit bekannt. Da diese Reaktionen bezüglich Ausbeuten und/oder Reaktionsbedingungen technisch oft unbefriedigend sind, wurde seit einiger Zeit auch die Verwendung von Edelmetallkatalysatoren untersucht.

In der Japanischen Kokai 81/15216 und in J. Amer. Chem. Soc. 102, 2096 (1980) werden Isomerisierungen von α,β-Epoxyketonen zu β-Diketonen in Gegenwart von Palladium(0)-Katalysatoren beschrieben. Epoxyketone mit einem α-Alkylsubstituenten reagieren jedoch nach dem angegebenen Verfahren im allgemeinen nur träge und geben nach relativ langen Reaktionszeiten nur geringe Ausbeuten an β-Diketon.

Es wurde nun gefunden, dass gemäss dem erfindungsgemässen Verfahren 1,3,3-Trimethyl-7-oxabicyclo[4.1.0]heptan-2-on mit beträchtlich kürzeren Reaktionszeiten und in deutlich besserer Ausbeute isomerisiert werden kann.

Das erfindungsgemässe Verfahren zur Herstellung von 3-Hydroxy-2,6,6-trimethyl-2-cyclohexen-1-on der Formel

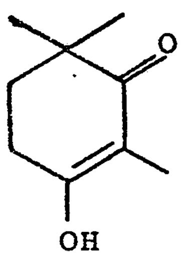

I

ist nun dadurch gekennzeichnet, dass man 1,3,3-Trimethyl-7-oxabicyclo[4.1.0]heptan-2-on der Formel

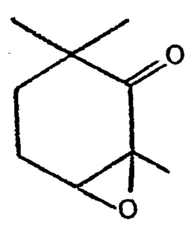

II

in Gegenwart von

a) einem Palladium(0)-Komplex eines Acetonderivates der allgemeinen Formel

$R^3$ O $R^4$
| || |
$R^1$-CH = C-C-C = CH-$R^2$ III

worin $R^1$ und $R^2$ Aryl bedeuten und $R^3$ und $R^4$ Wasserstoff oder einwertige, gegebenenfalls eine Carbonylfunktion aufweisende Kohlenwasserstoffreste bezeichnen,

b) einem Bis-(diphenylphosphino)-Derivat der allgemeinen Formel

$(R^5)_2P-R^6-P(R^5)_2$ IV

worin $R^5$ Phenyl oder Tolyl bezeichnet und $R^6$ eine gegebenenfalls mit einem oder mehreren inerten organischen Resten substituierte $C_2$–$C_4$-Alkylengruppe darstellt, und

c) einem Triarylphosphin der allgemeinen Formel

$P(R^7)_3$ V

worin $R^7$ einen gegebenenfalls alkylsubstituierten Phenylrest bedeutet, isomerisiert.

Die Verbindung der Formel I liegt im allgemeinen vorwiegend in der oben angegebenen Form vor. In der Literatur wird für diese Verbindung gelegentlich auch das tautomere 2,4,4-Trimethyl-1,3-cyclohexandion angegeben.

Die Reste $R^3$ und $R^4$ in obiger Formel III bezeichnen Wasserstoff oder einwertige Kohlenwasserstoffreste, wie Alkyl, Alkenyl, Aryl, Arylalkyl, Arylalkenyl etc., welche gegebenenfalls zusätzlich eine Carbonylfunktion aufweisen. Vorzugsweise steht eine gegebenenfalls vorhandene Carbonylfunktion in 1-Stellung des Restes. Besonders bevorzugte derartige Reste sind die Arylacrylyl-Reste. Vorzugsweise steht mindestens einer der Reste $R^3$ und $R^4$ für Wasserstoff. Besonders bevorzugte Liganden der Formel III sind somit diejenigen, worin $R^4$ Wasserstoff und $R^3$ Wasserstoff oder Arylacrylyl, insbesondere Wasserstoff, bezeichnen.

Der Ausdruck «Aryl» bedeutet im Rahmen der vorliegenden Erfindung (und insbesondere im Zusammenhang mit den Resten $R^1$–$R^4$ in Formel III) aromatische, carbocyclische Reste, wie Phenyl, Naphthyl und dergleichen, welche unsubstituiert oder mit inerten organischen Resten, wie Alkyl, Alkenyl, Hydroxy, Chlor etc., substituiert sein können. Bevorzugte Arylgruppen sind Tolyl und insbesondere Phenyl.

Beispiele bevorzugter Liganden der Formel III sind Dibenzylidenaceton und Tribenzylidenacetylaceton. Beispiele bevorzugter Palladium(0)-Komplexe sind dementsprechend Bis-(dibenzylidenaceton)palladium(0) und Tris-(tribenzylidenacetylaceton)-dipalladium(0)-Chloroform.

Die Gruppe $R^6$ in Formel IV bezeichnet eine $C_2$–$C_4$-Alkylengruppe, welche gegebenenfalls mit einem oder mehreren inerten organischen Resten, wie Alkyl, Alkenyl, Hydroxy, Chlor etc., vorzugsweise Methyl, substituiert ist. Bevorzugte Alkylengruppen sind diejenigen mit 2 oder 3 Kohlenstoffatomen, insbesondere Äthylen. Bevorzugter Rest $R^5$ ist Phenyl. Besonders bevorzugtes Bis-(diphenylphosphino)-Derivat ist somit 1,2-Bis-(diphenylphosphino)-äthan.

Die Reste $R^7$ in Formel V bedeuten vorzugsweise Gruppen mit 6 bis 10 Kohlenstoffatomen, wie Phenyl, Tolyl, Xylyl und dergleichen. Besonders bevorzugtes Triarylphosphin ist Triphenylphosphin.

Die erfindungsgemässe Isomerisierung der Verbindung der Formel II kann in einem inerten organischen Lösungsmittel, beispielsweise einem Kohlenwasserstoff, wie Toluol, Xylol, Decalin und dergleichen, durchgeführt werden. Vorzugsweise wird die Isomerisierung jedoch ohne Lösungsmittel durchgeführt. Temperatur und Druck sind keine kritischen Aspekte in dieser Reaktion. Im allgemeinen wird jedoch bei einer Temperatur von etwa 80–200°C gearbeitet. Bevorzugte Temperaturbereiche sind 120–160°C und insbesondere 140–150°C.

Das erfindungsgemässe Verfahren kann in Gegenwart katalytischer Menge an Palladium(0)-Komplex, Bis-(diphenylphosphino)-Derivat und Triarylphosphin erfolgen. Die Menge an Palladium(0)-Komplex liegt jedoch mit Vorteil bei etwa 1–10 Mol-%, insbesondere etwa 3–8 Mol-% bezogen auf das Ausgangsmaterial der Formel II. Der Anteil an Bis-(diphenylphosphino)-Derivat der Formel IV beträgt im allgemeinen etwa 0,5–5 Molaquivalente, vorzugsweise etwa 1–3 Moläquivalente bezogen auf eingesetztes Palladium. Der Anteil an Triarylphosphin der Formal V liegt üblicherweise bei etwa 0,5–5 Moläquivalenten, vorzugsweise etwa 1–3 Moläquivalenten bezogen auf eingesetztes Palladium. Die angegebenen Konzentrationsbereiche sind jedoch nicht kritisch und insbesondere können auch höhere Mengen an Palladium(0)-Komplex und Verbindungen der Formel IV und V verwendet werden.

Die Aufarbeitung des Reaktionsgemisches kann in an sich bekannter Weise erfolgen. Vorzugsweise wird mittels Lauge, z.B. Natronlauge, das entsprechende Enolat der Verbindung der Formel I aus dem Gemisch extrahiert. Auf diese Weise wird mit geringem Reinigungsaufwand direkt ein Produkt hoher Reinheit erhalten.

Die Verbindungen der Formeln I–V und die Palladium-(0)-Komplexe der Verbindungen der Formel III sind bekannte oder Analoge bekannter Verbindungen und können nach bekannten Methoden hergestellt werden.

Die weitere Umsetzung der Verbindung der Formel I zu Canthaxanthin kann in an sich bekannter Weise, z.B. nach dem in der Deutschen Offenlegungsschrift 2625259 beschriebenen Verfahren erfolgen.

Das erfindungsgemässe Verfahren wird durch das folgende Beispiel weiter veranschaulicht.

Beispiel 1

In einem Rundkolben, versehen mit Thermometer und Rückflusskühler, wurden unter Argonbegasung 15,4 g 1,3,3-Trimethyl-7-oxabicyclo[4.1.0]heptan-2-on, 1,8 g Triphenylphosphin und 2,76 g 1,2-Bis-(diphenylphosphino)-äthan vorgelegt und das Gemisch mit 2,0 g Bis-(dibenzylidenaceton)-palladium(0) versetzt. Dann wurde das Reaktionsgefäss in ein auf 150°C vorgeheiztes Ölbad getaucht und das Reaktionsgemisch bei dieser Temperatur unter fortgesetzter Argonbegasung während 1 Stunde mit einem Magnetrührer gerührt. Eine gaschromatographische Analyse des erhaltenen Gemisches ohne internen Standard ergab Peaks für 3-Hydroxy-2,6,6-trimethyl-2-cyclohexen-1-on (81,9 Flächen-%) und 2,6,6-Trimethyl-2-cyclohexen-1-on (18 Flächen-%). Zur Aufarbeitung wurde das auf Raumtemperatur abgekühlte Reaktionsgemisch in 20 ml Essigsäureäthylester aufgenommen und in einer Glassinternutsche über Kieselgel filtriert. Der Nutscheninhalt wurde noch dreimal mit je 150 ml Essigsäureäthylester ausgewaschen. Das Filtrat wurde im Rotationsverdampfer am Wasserstrahlvakuum bei einer Badtemperatur von 40°C bis zur Gewichtskonstanz eingedampft. Der Rückstand (21,5 g gelbliches Öl) wurde in 100 ml Diäthyläther gelöst und die Lösung in einen Scheidetrichter $S_1$ gegeben. Zwei weitere Scheidetrichter $S_2$ und $S_3$ wurden mit je 100 ml Diäthyläther beschickt. Dann wurden dreimal je 150 ml 3N Natronlauge der Reihe nach und unter jeweils guter Durchmischung durch die drei Extraktionsgefässe $S_1$–$S_3$ geschickt. Die Ätherphasen wurden verworfen. Die vereinigten alkalischen Extrakte wurden mit ca. 300 ml konzentrierter Salzsäure unter Kühlung mit Eis vorsichtig angesäuert (pH ca. 1) und dann unter Rühren mit 500 g Kochsalz gesättigt. Drei Scheidetrichter $S_4$–$S_6$ wurden mit je 200 ml Essigsäureäthylester beschickt. Danach wurden die wässrige Produktlösung und anschliessend dreimal je 150 ml gesättigte Kochsalzlösung der Reihe nach und unter jeweils guter Durchmischung durch die drei Extraktionsgefässe $S_4$–$S_6$ geschickt. Die vereinigten organischen Phasen wurden über Natriumsulfat getrocknet. Das Trocknungsmittel wurde abgenutscht und auf der Nutsche zweimal mit je 100 ml Essigsäureäthylester nachgewaschen. Das Filtrat wurde im Rotationsverdampfer am Wasserstrahlvakuum bei einer Badtemperatur von 40°C eingedampft und der Rückstand am Hochvakuum grtrocknet. Es resultierten 10,1 g (65,6%) 3-Hydroxy-2,6,6-trimethyl-2-cyclohexen-1-on als farblose Kristalle mit einer Reinheit von 99,6%; Smp. 112–114°C nach Umkristallisation aus Diisopropyläther.

## Patentansprüche

1. Verfahren zur Herstellung von 3-Hydroxy-2,6,6-trimethyl-2-cyclohexen-1-on der Formel

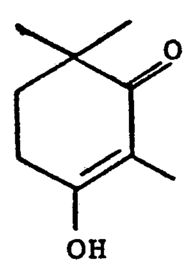

I

dadurch gekennzeichnet, dass man 1,3,3-Trimethyl-7-oxabicyclo[4.1.0]heptan-2-on der Formel

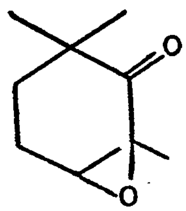

II

in Gegenwart von

a) einem Palladium(0)-Komplex eines Acetonderivates der allgemeinen Formel

$$\begin{array}{c} \quad R^3\ O\ R^4 \\ \quad |\ \ \|\ \ | \\ R^1\text{-}CH = C\text{-}C\text{-}C = CH\text{-}R^2 \end{array} \qquad III$$

worin $R^1$ und $R^2$ Aryl bedeuten und $R^3$ und $R^4$ Wasserstoff oder einwertige, gegebenenfalls eine Carbonylfunktion aufweisende Kohlenwasserstoffreste bezeichnen,

b) einem Bis-(diphenylphosphino)-Derivat der allgemeinen Formel

$$(R^5)_2P\text{-}R^6\text{-}P(R^5)_2 \qquad IV$$

worin $R^5$ Phenyl oder Tolyl bezeichnet und $R^6$ eine gegebenenfalls mit einem oder mehreren inerten organischen Resten substituierte $C_2$-$C_4$-Alkylengruppe darstellt, und

c) einem Triarylphosphin der allgemeinen Formel

$$P(R^7)_3 \qquad V$$

worin $R^7$ einen gegebenenfalls alkylsubstituierten Phenylrest bedeutet, isomerisiert.

2. Verfahren nach Anspruch 1, dadurch gekennzeichnet, dass man die Isomerisierung bei einer Temperatur von 80–200°C, vorzugsweise 120–160°C, durchführt.

3. Verfahren nach Anspruch 1 oder 2, dadurch gekennzeichnet, dass man die Isomerisierung in Gegenwart eines Palladium(0)-Komplexes eines Acetonderivates der Formel III durchführt, worin $R^3$ und $R^4$ Wasserstoff bedeuten.

4. Verfahren nach einem der Ansprüche 1 bis 3, dadurch gekennzeichnet, dass man die Isomerisierung in Gegenwart eines Palladium(0)-Komplexes eines Acetonderivates der Formel III durchführt, worin $R^1$ und $R^2$ Phenyl oder Tolyl, vorzugweise Phenyl bedeuten.

5. Verfahren nach einem der Ansprüche 1 bis 4, dadurch gekennzeichnet, dass man die Isomerisierung in Gegenwart von Bis-(dibenzylidenaceton)palladium(0) durchführt.

6. Verfahren nach einem der Ansprüche 1 bis 5, dadurch gekennzeichnet, dass man die Isomerisierung in Gegenwart eines Bis-(diphenylphosphino)-Derivates der Formel IV durchführt, worin $R^6$ Äthylen bedeutet.

7. Verfahren nach einem der Ansprüche 1 bis 6, dadurch gekennzeichnet, dass man die Isomerisierung in Gegenwart eines Bis-(diphenylphosphino)-Derivates der Formel IV durchführt, worin $R^5$ Phenyl bedeutet.

8. Verfahren nach einem der Ansprüche 1 bis 7, dadurch gekennzeichnet, dass man die Isomerisierung in Gegenwart eines Triarylphosphins der Formel V durchführt, worin $R^7$ Phenyl oder Tolyl, vorzugsweise Phenyl bedeutet.

9. Verfahren nach einem der Ansprüche 1 bis 8, dadurch gekennzeichnet, dass man die Reaktion in Gegenwart von 1–10 Mol-%, vorzugsweise 3–8 Mol-% an Palladium(0)-Komplex bezogen auf das Ausgangsmaterial der Formel II durchführt.

10. Verfahren nach einem der Ansprüche 1 bis 9, dadurch gekennzeichnet, dass man die Reaktion in Gegenwart von je 0,5–5 Moläquivalenten, vorzugsweise je 1–3 Moläquivalenten an Bis-(diphenylphosphino)-Derivat der Formel IV und an Triarylphosphin der Formel V bezogen auf eingesetztes Palladium durchführt.

## Claims

1. A process for the manufacture of 3-hydroxy-2,6,6-trimethyl-2-cyclohexen-1-one of the formula

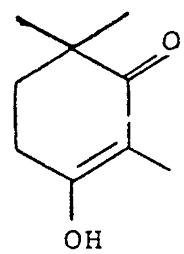

I

characterized by isomerizing 1,3,3-trimethyl-7-oxabicyclo[4.1.0]heptan-2-one of the formula

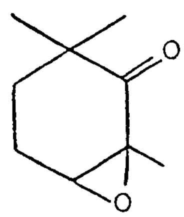

II

in the presence of

(a) a palladium(0) complex of an acetone derivative of the general formula

$$\begin{array}{c} \quad R^3\ O\ R^4 \\ \quad |\ \ \|\ \ | \\ R^1\text{-}CH = C\text{-}C\text{-}C = CH\text{-}R^2 \end{array} \qquad III$$

wherein $R^1$ and $R^2$ signify aryl and $R^3$ and $R^4$ denote hydrogen or monovalent hydrocarbon residues optionally carrying a carbonyl function,

(b) a bis-(diphenylphosphino) derivative of the general formula

$$(R^5)_2P\text{-}R^6\text{-}P(R^5)_2 \qquad IV$$

wherein $R^5$ denotes phenyl or tolyl and $R^6$ represents a $C_2$-$C_4$-alkylene group optionally substituted with one or more inert organic residues, and

c) a triarylphosphine of the general formula

$P(R^7)_3$ V

wherein $R^7$ signifies an optionally alkyl-substituted phenyl residue.

2. A process according to claim 1, characterized in that the isomerization is carried out at a temperature of 80–200°C, preferably 120–160°C.

3. A process according to claim 1 or 2, characterized in that the isomerization is carried out in the presence of a palladium(0) complex of an acetone derivative of formula III in which $R^3$ and $R^4$ signify hydrogen.

4. A process according to any one of claims 1 to 3, characterized in that the isomerization is carried out in the presence of a palladium(0) complex of an acetone derivative of formula III in which $R^1$ and $R^2$ signify phenyl or tolyl, preferably phenyl.

5. A process according to any one of claims 1 to 4, characterized in that the isomerization is carried out in the presence of bis-(dibenzylidene acetone)palladium(0).

6. A process according to any one of claims 1–5, characterized in that the isomerization is carried out in the presence of a bis-(diphenylphosphino) derivative of formula IV in which $R^6$ signifies ethylene.

7. A process according to any one of claims 1 to 6, characterized in that the isomerization is carried out in the presence of a bis-(diphenylphosphino) derivative of formula IV in which $R^5$ signifies phenyl.

8. A process according to any one of claims 1 to 7, characterized in that the isomerization is carried out in the presence of a triarylphosphine of formula V in which $R^7$ signifies phenyl or tolyl, preferably phenyl.

9. A process according to any one of claims 1 to 8, characterized in that the reaction is carried out in the presence of 1–10 mol-%, preferably 3–8 mol-%, of palladium(0) complex based on the starting material of formula II.

10. A process according to any one of claims 1 to 9, characterized in that the reaction is carried out in the presence of in each case 0.5–5 mol equivalents, preferably in each case 1–3 mol equivalents, of a bis-(diphenylphosphino) derivative of formula IV and of a triarylphosphine of formula V based on palladium used.

**Revendications**

1. Procédé de préparation de 3-hydroxy-2,6,6-triméthyl-2-cyclohexène-1-one de formule

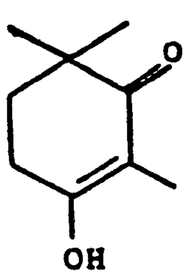

I

caractérisé en ce qu'on isomérise la 1,3,3-triméthyl-7-oxabicyclo[4.1.0]heptan-2-one de formule

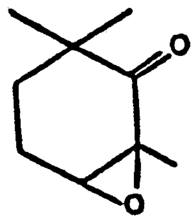

II

en présence de

a) un complexe de palladium(0) d'un dérivé d'acétone de formule générale

$R^3$ O $R^4$
| ‖ |
$R^1$-CH = C-C-C = CH-$R^2$ III

où $R^1$ et $R^2$ représentent un aryle et $R^3$ et $R^4$ un hydrogène ou des radicaux hydrocarbonés monovalents présentant éventuellement une fonction carbonyle,

b) un dérivé bis-(diphénylphosphino) de formule générale

$(R^5)_2P-R^6-P(R^5)_2$ IV

où $R^5$ représente un phényle ou un tolyle et $R^6$ un groupe alcoylène en $C_2$ à$C_4$ éventuellement substitué par un ou plusieurs radicaux organiques inertes, et

c) une triarylphosphine de formule générale

$P(R^7)_3$ V

où $R^7$ représente un radical phényle éventuellement substitué par un alcoyle.

2. Procédé selon la revendication 1, caractérisé en ce qu'on conduit l'isomérisation à une température de 80–200°C, de préférence 120–160°C.

3. Procédé selon la revendication 1 ou 2, caractérisé en ce qu'on conduit l'isomériation en présence d'un complexe de palladium(0) d'un dérivé d'acétone de formule III où $R^3$ et $R^4$ représentent un hydrogène.

4. Procédé selon l'une des revendications 1 à 3, caractérisé en ce qu'on conduit l'isomérisation en présence d'un complexe de palladium(0) d'un dérivé d'acétone de formule III où $R^1$ et $R^2$ représentent un phényle ou un tolyle, de préférence un phényle.

5. Procédé selon l'une des revendications 1 à 4, caractérisé en ce qu'on conduit l'isomérisation en présence de bis-(dibenzylidèneacétone)palladium(0).

6. Procédé selon l'une des revendications 1 à 5, caractérisé en ce qu'on conduit l'isomérisation en présence d'un dérivé bis-(diphénylphosphino) de formule IV où $R^6$ représente un éthylène.

7. Procédé selon l'une des revendications 1 à 6, caractérisé en ce qu'on conduit l'isomérisation en présence d'un dérivé de bis-(diphénylphos-

phino) de formule IV où $R^5$ représente un phényle.

8. Procédé selon l'une des revendications 1 à 7, caractérisé en ce qu'on conduit l'isomérisation en présence d'une triarylphosphine de formule V où $R^7$ représente un phényle ou un tolyle, de préférence un phényle.

9. Procédé selon l'une des revendications 1 à 8, caractérisé en ce qu'on conduit la réaction en présence de 1–10% molaire, de préférence 3–8% molaire, de complexe de palladium(0) par rapport au produit de départ de formule II.

10. Procédé selon l'une des revendications 1 à 9, caractérisé en ce qu'on conduit la réaction en présence respectivement de 0,5–5 équivalents molaires, de préférence respectivement de 1–3 équivalents molaires, de dérivé bis-(diphénylphosphino) de formule IV et de triarylphosphine de formule V par rapport au palladium utilisé.